# EUROPÄISCHE PATENTSCHRIFT

(11) **EP 1 105 035 B1**
(45) Veröffentlichungstag und Bekanntmachung des Hinweises auf die Patenterteilung: **27.10.2004**
(21) Anmeldenummer: 99944467.2
(22) Anmeldetag: 20.08.1999
(51) Int. Cl.: A61B 1/005

(54) **SEITENBLICK-ENDOSKOP**
LATERAL-VIEWING ENDOSCOPE
ENDOSCOPE A VISION LATERALE

(30) Priorität: 28.08.1998 DE 19839188
(43) Veröffentlichungstag der Anmeldung: 13.06.2001
(73) Patentinhaber: Storz-Endoskop GmbH, 8200 Schaffhausen (CH)
(72) Erfinder: KRATTIGER, Beat, CH-8222 Beringen (CH); HAAN, Harald, CH-8200 Schaffhausen (CH); KUSTER, Manfred, CH-8200 Schaffhausen (CH); NOVAK, Pavel, CH-8207 Schaffhausen (CH); RELING, Jörg, D-78604 Rietheim/Weilheim (DE)
(74) Vertreter: Heuckeroth, Volker, Dr.
(86) Internationale Anmeldenummer: PCT/EP1999/006142
(87) Internationale Veröffentlichungsnummer: WO 2000/011999

(56) Entgegenhaltungen:
- EP-A- 0 369 937
- EP-A- 0 682 910
- DE-C- 190 703
- GB-A- 2 138 687
- US-A- 4 874 371
- US-A- 5 702 349
- US-A- 5 762 603

## Beschreibung

Die Erfindung betrifft ein Endoskop, mit einem Schaft, mit einem proximal angeordneten seitlich von dem Schaft abstehenden pistolenartigen Handgriff, mit einem Mechanismus zur Verschwenkung einer Blickrichtung in einen Beobachtungsraum, und mit zumindest zwei mit dem Mechanismus in Wirkverbindung stehenden Bedienelementen zur Betätigung des Mechanismus, wobei das zumindest eine Bedienelement zur Verschwenkung der Blickrichtung bezüglich der Längsrichtung des Schafts und das zumindest eine weitere Bedienelement zur Verschwenkung der Blickrichtung in Umfangsrichtung des Schafts dient, wobei das eine Bedienelement an einer Vorderseite des Handgriffs so angeordnet ist, daß es mit zumindest einem Finger der Hand, die den Handgriff hält, betätigbar ist.

Ein derartiges Endoskop ist aus der GB-A-2 138 687 bekannt.

Endoskope gewinnen sowohl in technischen als auch in medizinischen Anwendungen zunehmend an Bedeutung. In technischen Bereichen ermöglichen solche Endoskope eine kostengünstige, zerstörungsfreie, visuelle Werkstoffprüfung. Mit einem Endoskop können schwer zugängliche Hohlräume und verdeckte Bauteile ohne zeitraubende Demontage visuell geprüft werden. Ein weiteres Anwendungsgebiet der technischen Endoskopie ist die Untersuchung von Verbrennungsvorgängen in Verbrennungsmotoren, Turbinen oder Reaktoren, um nur einige Beispiele zu nennen.

Auf medizinischem Gebiet werden Endoskope in der minimalinvasiven Chirurgie eingesetzt. Mit Hilfe von Endoskopen können Operationen ohne die Schaffung größerer Operationsöffnungen unter Sichtkontrolle durch das Endoskop mit geringerer Belastung des Patienten durchgeführt werden.

Bei einer einfachen Art von bekannten Endoskopen ist die Blickrichtung, unter der man die Richtung der Symmetrieachse des durch den Bildwinkel begrenzten Lichtkegels des in das distale Ende des Schafts einfallenden Beobachtungslichts versteht, fest vorgegeben und - außer durch Lageveränderung des Endoskops, die jedoch nur sehr begrenzt möglich ist - unveränderbar. Es wurden daher, um ein Beobachtungsobjekt unter verschiedenen Blickrichtungen beobachten zu können, oder um den beobachtbaren Raum, d.h. das Blickfeld, zu vergrößern, separate Endoskope mit unterschiedlichen Optiken geschaffen, bspw. mit Geradeaus-, Schrägvoraus-, Seit-, und Rückblick-Optik. Um die Blickrichtung zu verändern, müssen bei dieser Art von Endoskopen die Endoskope während eines Einsatzes demnach gegeneinander ausgetauscht werden, was jedoch umständlich ist, da bei jedem Wechsel das eine Endoskop aus der Einführöffnung, durch die das Endoskop in den Beobachtungsraum eingeführt ist, herausgezogen und das nächste Endoskop eingeführt werden muß.

Um mit einem einzigen Endoskop ein Beobachtungsobjekt unter verschiedenen Blickrichtungen inspizieren zu können oder im Beobachtungsraum mit dem Endoskop einen größeren Bereich einsehen zu können, wurden Endoskope entwickelt, die einen Mechanismus zum Verschwenken der Blickrichtung, zumindest in Umfangsrichtung des Schafts, aufweisen. Die Blickrichtung kann damit ohne Lageveränderung des Endoskops verändert werden.

Das aus der eingangs genannten GB-A-2 138 687 bekannte Endoskop weist einen Mechanismus sowohl zur Verschwenkung der Blickrichtung durch das Endoskop bezüglich der Längsrichtung des Schafts als auch zur Verschwenkung der Blickrichtung in Umfangsrichtung des Schafts auf. Als Bedienelement zur Verschwenkung der Blickrichtung bezüglich der Längsrichtung des Schafts ist vor dem seitlich von dem Schaft abstehenden pistolenartigen Handgriff ein Bedienelement mit einem Fingerring angeordnet. Dieses Bedienelement dient zum Verschwenken der Blickrichtung bezüglich der Längsachse des Schafts. Ein weiteres Bedienelement zum Verschwenken der Blickrichtung in Umfangsrichtung des Schafts durch Drehen des Schafts ist noch vor dem zuvor genannten Bedienelement angeordnet und besteht aus einem quer zum Endoskopgehäuse zur selben Seite wie der Handgriff abstehenden Hebel, der sich in Umfangsrichtung, d.h. quer zur Längsachse des Endoskopgehäuses, verschwenken läßt. Dieses weitere Bedienelement ist jedoch von dem vorgenannten ersten Bedienelement sehr weit beabstandet, so daß eine Einhandbedienung des Endoskops nicht möglich ist, wenn beide zuvor genannten Bedienelemente gleichzeitig betätigt werden sollen.

Ein weiteres Endoskop ist aus der DE-Firmenschrift der OLYMPUS OPTICAL CO. (EUROPA) GmbH, Hamburg, "OLYMPUS - THE VISIBLE DIF-FERENCE" unter der Bezeichnung "Schwenkprisma-Boreskop" bekannt.

Dieses bekannte Endoskop weist am proximalen Ende einen seitlich vom Schaft des Endoskops abstehenden Handgriff auf. Ein solcher Handgriff läßt sich bequem in der Hand halten, da ein solcher Handgriff in der Hand liegend mit den Fingern und dem Daumen umgriffen werden kann. Ferner ist ein Bedienelement vorgesehen, das mit dem Mechanismus zur Verschwenkung der Blickrichtung in Wirkverbindung steht, so daß über eine Betätigung des Bedienelementes die jeweils gewünschte Blickrichtung eingestellt werden kann.

Bei diesem bekannten Endoskop ist das Bedienelement als Drehring ausgebildet, der konzentrisch zur Schaftachse in der Nähe des Okulars angeordnet ist. Diese Ausgestaltung des Endoskops ist hinsichtlich seiner Bedienungseigenschaften nachteilig. Wird nämlich dieses Endoskop mit einer Hand gehalten, muß zur Verschwenkung der Blickrichtung das Bedienelement mit der anderen Hand betätigt werden. Es ist nämlich nicht oder nur erschwert möglich, den Drehring mit derselben Hand zu bedienen, die den Handgriff greift, da der Drehring über und seitlich hinter dem Handgriff positioniert ist. Außerdem erfordert ein derartiger Drehring zu seiner Betätigung in der Regel eine Bedienung mittels Daumen und Zeigefinger. Es kann nicht gleichzeitig die Blickrichtung verschwenkt und das Bild fokussiert werden, da zur Betätigung des Fokussierrings keine Hand mehr frei ist, wenn gerade das Bedienelement zur Verschwenkung der Blickrichtung betätigt wird. Da der Drehring bezüglich des Handgriffs in nicht ergonomischer Weise positioniert und als zum Schaft konzentrischer Drehring ausgebildet ist, kann das Endoskop nicht gleichzeitig mit einer Hand gehalten und mit derselben Hand der Drehring betätigt werden, es eignet sich somit nicht für eine Einhand-Bedienung.

Der Erfindung liegt daher die Aufgabe zugrunde, ein Endoskop der eingangs genannten Art dahingehend weiterzubilden, daß die Bedienungseigenschaften des Endoskops verbessert werden.

Erfindungsgemäß wird diese Aufgabe hinsichtlich des eingangs genannten Endoskops dadurch gelöst, daß das zumindest eine andere Bedienelement mit dem Daumen derselben Hand betätigbar an einer Seitenfläche des Handgriffs angeordnet ist.

Dadurch, daß beide Bedienelemente am Handgriff selbst derart positioniert sind, daß sie mittels zumindest eines Fingers und des Daumens derselben Hand, die den Handgriff hält, betätigbar sind, ermöglicht das erfindungsgemäße Endoskop eine Einhand-Bedienung, die bei dem bekannten Endoskop nicht gegeben ist. Die Positionierung der Bedienelemente am Handgriff ermöglicht es einerseits, während der Betätigung eines oder beider Bedienelemente den Handgriff weiter fest in der Hand zu halten, und andererseits, das Bedienelement ohne wesentliche Lageänderung der Hand oder durch Umgreifen betätigen zu können. Der seitlich abstehende Handgriff liegt also auch bei Betätigung beider Bedienelemente weiter bequem in der Hand. Die Erfindung besteht somit darin, beide Bedienelemente an dem Handgriff an einer Stelle zu positionieren, an der bei normaler entspannter Handhaltung der Daumen und zumindest einer der Finger beim Greifen des Handgriffs zu liegen kommt. Dadurch wird vorteilhafterweise erreicht, daß sich die Bedienelemente durch geringfügige Finger- oder Daumenbewegungen betätigen lassen. Das erfindungsgemäße Endoskop ist aufgrund seiner Einhand-Bedienbarkeit bezüglich seiner Bedienungsfreundlichkeit erheblich verbessert.

Die Positionierung des zumindest einen Bedienelements an der Vorderseite des Handgriffs hat den Vorteil, daß das Bedienelement mit den Fingerkuppen oder den vorderen Fingergliedern eines oder mehrerer Finger derselben Hand, die den Handgriff greift, betätigt werden kann. Ein weiterer Vorteil besteht darin, daß das Endoskop von Links-, wie von Rechtshändern gleichermaßen bedient werden kann, weil sich das Bedienelement an der distalen Seite des Handgriffs etwa mittig positionieren läßt.

Bei dem erfindungsgemäßen Endoskop sind zwei Bedienelemente vorgesehen, wobei das zumindest eine Bedienelement zur Verschwenkung der Blickrichtung bezüglich der Längsrichtung und das zumindest eine weitere Bedienelement zur Verschwenkung der Blickrichtung in Umfangsrichtung derart zueinander positioniert sind, daß eines der Bedienelemente mit dem Daumen und das andere mit zumindest einem Finger derselben Hand, die den Handgriff hält, betätigbar ist.

Bei dieser Ausgestaltung wird durch Lageverstellung des optischen Elements die Blickrichtung bezüglich der Längsrichtung des Schafts verschwenkt, d.h. durch Lageverstellung des optischen Elements wird die Blickrichtung relativ zur Längsachse des Schafts in einem Winkelbereich zwischen bspw. einer Vorwärts-Blickrichtung und einer Rückwärts-Blickrichtung verstellt. Durch Verdrehen des Schafts wird dagegen die Blickrichtung in Umfangsrichtung des Schafts verändert. Auch das aus der Firmenschrift bekannte Endoskop weist zwar einen drehbaren Schaft und ein verschwenkbares Prisma auf. Sowohl zur Verdrehung des Schafts als auch zur Verschwenkung des Prismas ist jedoch jeweils ein konzentrisch um den Schaft angeordneter Drehring vorgesehen, der sich, wie bereits eingangs erwähnt, nicht mit derselben Hand bedienen läßt, die den Handgriff hält. Demgegenüber wird bei der erfindungsgemäßen Ausgestaltung eine Einhand-Bedienung für zwei Bedienelemente ermöglicht, weil diese Bedienelemente am Handgriff ergonomisch positioniert sind.

Der weitere erhebliche Vorteil dieser Maßnahme besteht darin, daß durch die Positionslage des Bedienelements zum Verdrehen des Schafts und des Bedienelements zur Lageverstellung des optischen Elements eine Bedienungsanordnung geschaffen wird, die ein gleichzeitiges Betätigen beider Bedienelemente ermöglicht. Das zumindest eine Bedienelement kann bspw. mit dem Zeigefinger und das weitere Bedienelement mit dem Daumen betätigt werden, wodurch die gewünschte Blickrichtung wesentlich einfacher und vor allem schneller eingestellt werden kann, was bei dem bekannten Endoskop ebenfalls nicht möglich ist.

Besonders bevorzugt ist es, wenn das zumindest eine Bedienelement als pistolenartiger Abzug ausgebildet ist.

Hierbei ist in Verbindung mit der Positionierung des Bedienelements an der Vorderseite des Handgriffs besonders vorteilhaft, daß ein solcher Abzug mit geringem Betätigungsweg und somit geringer Fingerbewegung durch Fingerkrümmung bedient werden kann. Der Abzug, der als Schwenkhebel ausgebildet sein kann, kann beispielsweise mit dem Zeigefinger betätigt werden, während die übrigen drei Finger und der Daumen den Handgriff weiter bequem festhalten können. Diese Ausgestaltung des Bedienelements ist daher besonders ergonomisch.

In einer weiteren bevorzugten Ausgestaltung ist das zumindest eine Bedienelement als Taste, Tastenpaar oder als Wipptaste ausgebildet.

Mit dieser Ausgestaltung wird ebenfalls vorteilhaft eine Einhand-Bedienung ermöglicht. Die Taste kann dabei an einer Seitenfläche des Handgriffs positioniert werden, so daß sie mit dem Daumen betätigt werden kann oder wie vorstehend erwähnt, an der Vorderseite des Handgriffs, so daß sie mit einem Finger der den Handgriff umgreifenden Hand betätigt werden kann. Bei der Ausgestaltung als Tastenpaar ist die eine Taste bevorzugt für die Verschwenkung der Blickrichtung in der einen Richtung und die benachbarte Taste für die Verschwenkung in Gegenrichtung ausgelegt. Bei der Ausgestaltung als Wipptaste ist die eine Tastenseite bevorzugt für die Verschwenkung des Blickes in eine Richtung, und die benachbarte Seite für die Verschwenkung in Gegenrichtung ausgelegt.

In einer weiteren bevorzugten Ausgestaltung weist das zumindest eine Bedienelement einen umfänglich geschlossenen oder umfänglich teilweise offenen Ring für eine Betätigung mittels eines oder mehrerer Finger auf.

Diese Maßnahme ist von Vorteil, wenn das Bedienelement nicht gegen eine Rückstellkraft beweglich ist, so daß das Bedienelement mit demselben oder denselben Fingern bzw. dem Daumen hin- und herbewegt werden kann, ohne daß der oder die Finger umgesetzt werden müssen.

In einer weiteren bevorzugten Ausgestaltung ist das zumindest eine andere Bedienelement als Schieber ausgebildet, der an der zumindest einen Seitenfläche des Handgriffs angeordnet ist.

Diese Maßnahme stellt eine weitere vorteilhafte Möglichkeit der Positionierung und Ausbildung des Bedienelements dar, die eine Betätigung mittels des Daumens allein ermöglicht. Wird auf beiden Seitenflächen des Handgriffs ein derartiger Schieber vorgesehen, kann das erfindungsgemäße Endoskop wiederum vorteilhaft mit der linken oder der rechten Hand in gleicher Weise bedient werden.

In einer weiteren bevorzugten Ausgestaltung ist das zumindest eine andere Bedienelement als Stellrad ausgebildet, das in dem Handgriff drehbar gelagert ist, wobei der Handgriff auf zumindest einer seiner Seitenflächen ein Fenster aufweist, in dem ein Teilumfang des Stellrads zu liegen kommt.

Hierbei ist von Vorteil, daß das Stellrad, von dem nur ein betätigbarer Teilumfang aus dem Handgriff vorsteht, mittels des Daumens mit geringen Daumenbewegungen gedreht werden kann. Bevorzugt ist es, wenn auf beiden Seitenflächen des Handgriffs jeweils ein betätigbarer Teilumfang des Stellrades zu liegen kommt, so daß das Endoskop sowohl links- als auch rechtshändig in gleicher Weise mit einer Hand bedient werden kann.

In einer weiteren bevorzugten Ausgestaltung ist das Stellrad mit nicht parallel zur Längsachse des Schafts verlaufender Drehachse in dem Handgriff angeordnet.

Hierbei ist von Vorteil, daß die Betätigungsrichtung des Stellrades etwa in Richtung der Längsachse des Schafts verläuft. Diese Betätigungsrichtung ist vorteilhaft an die Bewegungsfreiheit des Daumens angepaßt, wenn der Handgriff von der Hand des Benutzers umgriffen wird.

In einer weiteren bevorzugten Ausgestaltung weist der Mechanismus zur Verschwenkung der Blickrichtung zumindest ein am distalen Ende des Schafts lageverstellbar angeordnetes optisches Element auf, wobei die Lageverstellung des optischen Elements der Verschwenkung der Blickrichtung im wesentlichen bezüglich der Längsrichtung des Schafts dient, und wobei das zumindest eine Bedienelement mit dem optischen Element in Wirkverbindung steht.

In Verbindung mit der erfindungsgemäßen Positionierung des zumindest einen Bedienelements am Handgriff wird eine vorteilhaft einfach in Einhand-Bedienung betätigbare Verschwenkung der Blickrichtung zumindest in Längsrichtung bspw. zwischen einer Vorausblick- und einer Rückblickrichtung ermöglicht.

In einer weiteren bevorzugten Ausgestaltung umfaßt der Mechanismus eine Verdrehbarkeit des Schafts, wobei der Schaft zur Verschwenkung der Blickrichtung in Umfangsrichtung des Schafts um seine Längsachse drehbar ist, wobei das zumindest eine andere Bedienelement mit dem Schaft zum Drehen des Schafts in Wirkverbindung steht.

In Verbindung mit der erfindungsgemäßen Positionierung des zumindest einen Bedienelements am Handgriff wird eine vorteilhaft einfach in Einhand-Bedienung betätigbare Verschwenkung der Blickrichtung zumindest in Umfangsrichtung ermöglicht.

In einer weiteren bevorzugten Ausgestaltung ist das zumindest eine und/oder das zumindest eine weitere Bedienelement selbsthemmend ausgebildet.

Diese Maßnahme hat den Vorteil, daß eine eingestellte Blickrichtung beibehalten wird, ohne daß dazu das eine und/oder das andere Bedienelement lagefest gehalten werden muß. Weiterhin wird dadurch vorteilhaft eine ungewollte Verstellung der Blickrichtung vermieden.

In einer weiteren bevorzugten Ausgestaltung ist das zumindest eine und/oder das zumindest eine weitere Bedienelement in zumindest einer Stellung arretierbar.

Hierbei ist von Vorteil, daß zumindest eine Blickrichtung, bevorzugt jedoch jede Blickrichtung, die mittels des bzw. der Bedienelemente einstellbar ist, unverstellbar arretiert werden kann, so daß nach einer Unterbrechung eines Inspektionsvorgangs die Inspektion mit unveränderter Blickrichtung wieder aufgenommen werden kann und die gewünschte Beobachtungsstelle somit im Beobachtungsraum sofort wiedergefunden werden kann. Die Arretierung des Bedienelements zum Verdrehen des Schafts wird bevorzugt durch eine einschaltbare Rastung des Bedienelements erreicht.

In einer weiteren bevorzugten Ausgestaltung ist zumindest eine Anzeigeeinrichtung zur Anzeige der jeweils eingestellten Blickrichtung vorgesehen.

Hierbei ist von Vorteil, daß der Benutzer des Endoskops anhand der eingestellten und als Winkel bezüglich einer festen Richtung, beispielsweise einer endoskopfesten Achse, angezeigten Blickrichtung die Lage eines Beobachtungsobjekts bezüglich anderer Beobachtungsobjekte oder Raumpunkten im Beobachtungsraum in der Art einer Peilung bestimmen und dokumentieren kann.

In einer weiteren bevorzugten Ausgestaltung ist die Anzeigeeinrichtung im Bildübertragungsweg des Endoskops angeordnet, so daß die eingestellte Blickrichtung in einem Sucherbild der Endoskopoptik angezeigt wird.

Hierbei ist von Vorteil, daß die eingestellte Blickrichtung besonders bequem beim Durchblicken durch das Endoskop festgestellt werden kann.

Dabei ist es bevorzugt, wenn die Anzeigeeinrichtung ein lochscheibenförmiges Element, das umfänglich verteilt Referenzmarkierungen aufweist, und einen relativ zu diesen um die Längsachse des Schafts umlaufenden Zeiger aufweist.

Dies ist eine vorteilhafte Ausgestaltung der Anzeigeeinrichtung zur Anzeige der Blickrichtung in Umfangsrichtung, bei der der Zeiger beim Drehen des Schafts beispielsweise diesem umläuft, während das scheibenförmige Element, bspw. in Form einer Kompaßrose, das die Referenzmarkierungen aufweist, beim Drehen des Schafts ortsfest bleibt. Das Element, das die Referenzmarkierungen aufweist, kann beispielsweise eine Blende im Bildübertragungsweg des Endoskops sein. Die Referenzmarkierungen können beispielsweise auch mit Gradzahlen beschriftet sein, beispielsweise in 30°-Schritten. Durch die gleichzeitige Anzeige der Markierungen und des Zeigers ist der Winkel zwischen Handgriff und der Blickrichtung in Umfangsrichtung zumindest abschätzbar und auch voreinstellbar. Bei beispielsweise senkrechter Haltung des Handgriffs, bei der die Vertikale als Bezugsachse dient, ist die Blickrichtung in Umfangsrichtung somit definiert. Auf diese Art kann vorteilhafterweise eine Beobachtungsstelle wiedergefunden werden, oder es kann die Beobachtungsrichtung, mindestens in Umfangsrichtung, abgeschätzt und dokumentiert werden.

Weiterhin ist bevorzugt, wenn die Anzeigeeinrichtung eine Flüssigkeitslibelle zur Anzeige der Lotrichtung aufweist.

Diese Maßnahme hat den weiteren Vorteil, daß durch die Flüssigkeitslibelle, wie sie bei einer Wasserwaage vorgesehen ist, auch die Lotrichtung während der Handhabung des Endoskops auch bei seitlich gekippt gehaltenem Handgriff stets definiert ist. Die Flüssigkeitslibelle kann an Stelle oder zusätzlich zu den zuvor erwähnten Referenzmarkierungen vorgesehen sein.

In einer weiteren bevorzugten Ausgestaltung umfaßt die Anzeigeeinrichtung eine Digital- oder Analoganzeige für die eingestellte Blickrichtung.

Durch diese Maßnahme wird vorteilhafterweise eine genauere Anzeige der jeweiligen eingestellten Blickrichtung ermöglicht, und zwar kann eine Anzeige für die Blickrichtung in Längsrichtung und/oder in Umfangsrichtung vorgesehen sein. Die Anzeige kann auch im Bildübertragungsweg der Endoskopoptik im Sucherbild sichtbar sein.

In einer weiteren bevorzugten Ausgestaltung umfaßt die Anzeigeeinrichtung ein oder mehrere am Handgriff angeordnete Anzeigefenster mit einer Digital- oder Analoganzeige für die eingestellte Blickrichtung.

Diese Maßnahme hat den Vorteil, daß die eingestellte Blickrichtung in Längsrichtung und/oder in Umfangsrichtung auch am Handgriff selbst, so daß der Betriebszustand des Endoskops bezüglich der eingestellten Blickrichtung jederzeit erkennbar ist.

In einer weiteren bevorzugten Ausgestaltung steht der Handgriff vom Schaft zum distalen Ende hin geneigt ab.

Durch diese Ausgestaltung des Handgriffs wird eine ideal ergonomische Form des Handgriffs erreicht, die an die bestimmungsgemäße Handhabung des Endoskops angepaßt ist. Bei einem bestimmungsgemäßen Einsatz des Endoskops wird dieses nämlich mit seiner Okularmuschel dicht an das Auge des Benutzers herangeführt, so daß der Unterarm nahe am Oberarm liegt. Aufgrund des zum distalen Ende hin geneigten Handgriffs kann der Handgriff nun mit geringfügig nach hinten und oben abgewinkeltem Handgelenk auf besonders entspannte, ermüdungsfreie Weise gehalten werden.

In einer weiteren bevorzugten Ausgestaltung ist im Handgriff versenkt ein Anschluß zum Anschließen eines Licht zuführenden Kabels angeordnet, wobei der Handgriff in Höhe des Anschlusses zumindest einseitig eine Öffnung aufweist.

Der im Handgriff versenkte Anschluß hat den Vorteil, daß das in der Regel durch eine Knickschutztülle versteifte Kabelende vollständig im Handgriff versenkt werden kann, so daß erst der flexible Teil des Lichtleitkabels aus dem Handgriff heraustritt. Dies hat den Vorteil, daß das versteifte Kabelende die Bedienung des Endoskops nicht behindert, wodurch die Bedienungseigenschaften des Endoskops weiter verbessert werden. Die zumindest einseitig vorgesehene Öffnung, bevorzugt jedoch beidseitig in Höhe des Anschlusses vorgesehenen Öffnungen ermöglichen eine bequeme Manipulation zum Befestigen und Lösen des Lichtkabelsteckers.

In einer weiteren bevorzugten Ausgestaltung ist am proximalen Ende des Endoskops eine TV-Kamera integriert.

Die Kamera kann anstelle des sonst üblichen Okulars am proximalen Ende des Handgriffs integriert sein, so daß der Beobachtungsraum vorteilhaft auf einem Monitor visualisiert werden kann.

Die vorstehend genannten Vorteile des erfindungsgemäßen Endoskops lassen sich vorteilhaft in der technischen Endoskopie, aber auch in medizinischen Anwendungen, insbesondere in der endoskopischen Chirurgie, nutzen.

Weitere Vorteile ergeben sich aus der nachfolgenden Beschreibung und der beigefügten Zeichnung.

Es versteht sich, daß die vorstehend genannten und nachstehend noch zu erläuternden Merkmale nicht nur in ihrer jeweils angegebenen Kombination, sondern auch in anderen Kombinationen oder in Alleinstellung verwendbar sind, ohne den Rahmen der vorliegenden Erfindung zu verlassen.

Ausgewählte Ausführungsbeispiele sind in der Zeichnung dargestellt und werden hiernach mit Bezug auf die Figuren näher beschrieben. Es zeigen:
- Fig. 1: eine Gesamtdarstellung eines erfindungsgemäßen Endoskops in einer Seitenansicht;
- Fig. 2: das distale Ende des Endoskops in Fig. 1 in perspektivischer vergrößerter Darstellung, wobei ein Außenschaft des Endoskops in Fig. 1 weggelassen wurde;
- Fig. 3: eine schematische Darstellung des Endoskops in Fig. 1 im Bereich des Handgriffs in vergrößertem Maßstab, wobei der Handgriff geöffnet ist;
- Fig. 4: eine schematische Darstellung einer im Sucherbild sichtbaren Anzeige der Blickrichtung in vergrößertem Maßstab;
- Fig. 5: einen Ausschnitt einer Variante der Anzeige;
- Fig. 6: ein weiteres Ausführungsbeispiel eines erfindungsgemäßen Endoskops; und
- Fig. 7: ein weiteres Ausführungsbeispiel eines erfindungsgemäßen Endoskops.

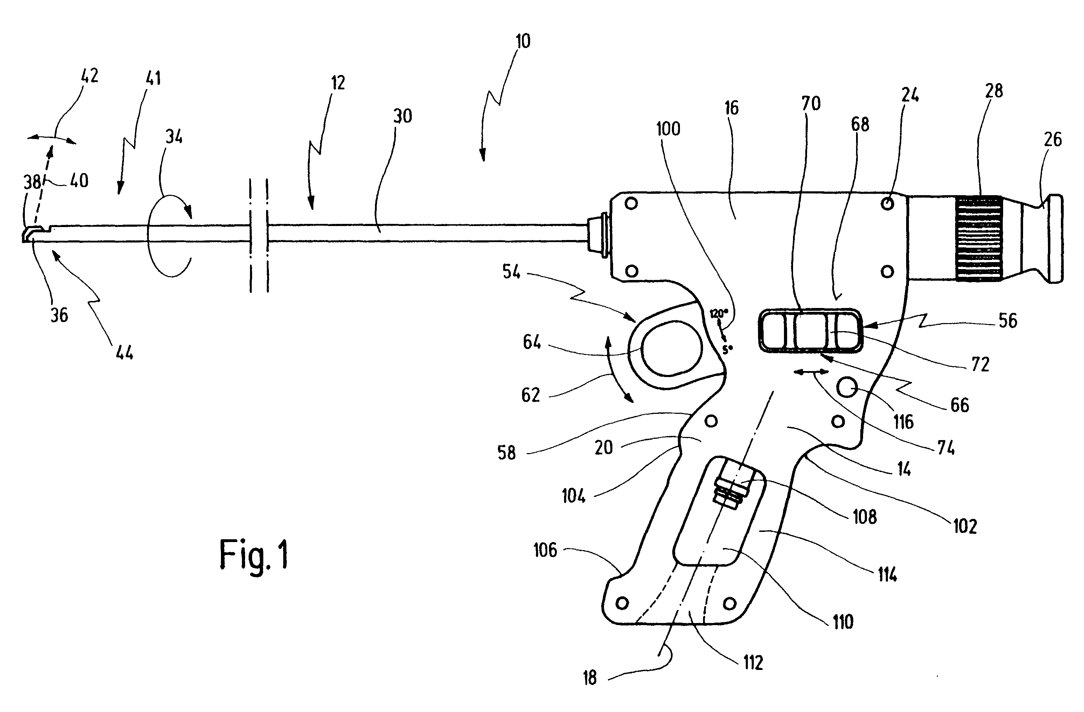

In Fig. 1 ist ein mit dem allgemeinen Bezugszeichen 10 versehenes Endoskop dargestellt. Das Endoskop 10 wird in der technischen Endoskopie zur Inspektion von Hohlräumen und darin enthaltenen Objekten verwendet, die für eine Beobachtung mit dem bloßen Auge nicht oder nur schwer zugänglich sind. Das Endoskop 10 kann jedoch auch für medizinische Anwendungen in der endoskopischen Chirurgie ausgelegt sein.

Das Endoskop 10 weist allgemein einen Schaft 12 und einen Handgriff 14 auf, der über ein einstückig mit dem Handgriff 14 verbundenes Gehäuse 16 am Schaft 12 befestigt ist. Der Handgriff 14 steht seitlich von dem Schaft 12 ab, und zwar derart, daß der Handgriff 14 vom Schaft 12 aus wegführend, zum distalen Ende hin geneigt ist. Die Neigungsrichtung des Handgriffs 14 ist durch eine Längsmittelachse 18 des Handgriffs 14 verdeutlicht.

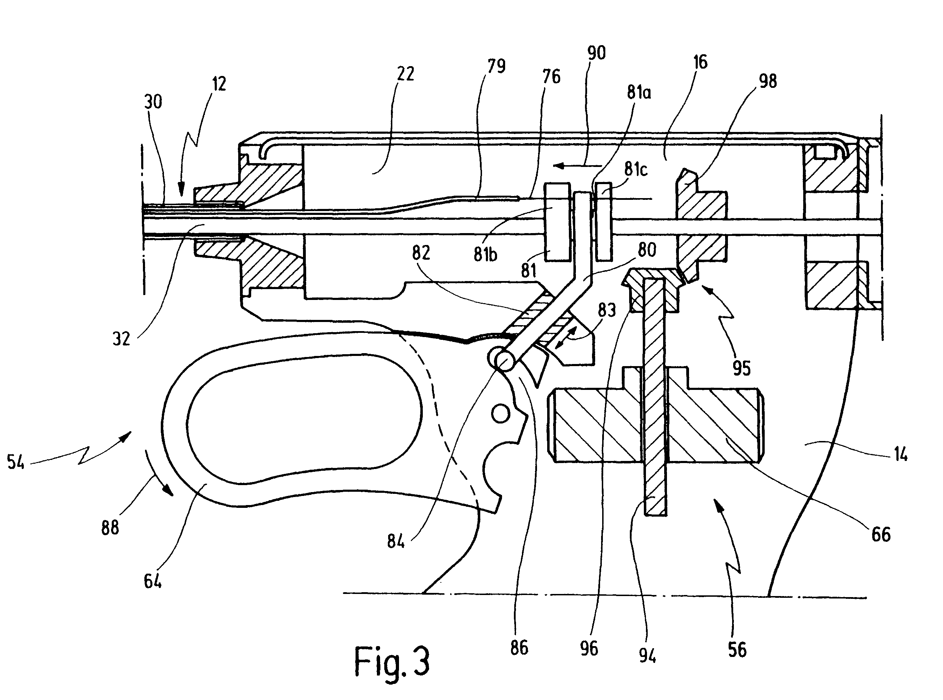

Der Handgriff 14 und das mit diesem einstückig verbundene Gehäuse 16 werden aus zwei Halbschalen 20 und 22 gebildet, von denen die linke Halbschale 20 in Fig. 1 und die rechte Halbschale 22 in Fig. 3 zu sehen ist. Die Halbschalen 20 und 22 sind an Befestigungsstellen 24 mittels Schrauben oder dergleichen aneinander befestigt.

Am proximalen Ende weist das Endoskop 10 eine Okularmuschel 26 auf, durch die das durch den Endoskopschaft 12 übertragene Bild des Beobachtungsgebietes mit dem Auge beobachtet werden kann. Ferner ist ein Okularfokussierring 28 vorgesehen, mit dem das beobachtete Bild scharfgestellt werden kann.

Der Schaft 12 wird durch einen Außenschaft 30 (vergl. Fig. 1 und 3) sowie einen in dem Außenschaft 30 angeordneten Innenschaft 32 gebildet. Der Innenschaft 32 nimmt das System der optisch abbildenden Elemente der Endoskopoptik auf, die in der Zeichnung nicht dargestellt sind, und die weiterhin in einem ebenfalls nicht dargestellten weiteren, in den Innenschaft 32 aufgenommenen Rohrschaft angeordnet sind.

Der Schaft 12, d.h. der Außenschaft 30 und der Innenschaft 32, sind zusammen um ihre Längsmittelachse relativ zu dem Handgriff 14 verdrehbar, wie mit einem Pfeil 34 angedeutet ist. Damit der Außenschaft 30 und der Innenschaft 32 gemeinsam verdrehbar sind, sind diese beiden in geeigneter Weise aneinander drehfest fixiert. An seinem distalen Ende 36 weist der Außenschaft 30 ein Fenster 38 in Form eines Kugelschalensegments auf, dessen Mittelpunkt nahe der nicht dargestellten Eintrittspupille liegt. Das Fenster 38 bildet die Lichteintrittsöffnung des Endoskopes 10. Der Beleuchtungsaustritt wird durch beidseits des Fensters 38 in den Schaft 12 eingelassene und polierte Lichtleiterenden gewährt.

Durch das Fenster 38 wird mit dem Endoskop 10 ein nicht dargestelltes Beobachtungsobjekt unter einer Blickrichtung 40 beobachtet. Die Blickrichtung 40 ist, wie hiernach noch näher beschrieben wird, über einen Mechanismus 41 verschwenkbar. Mittels des Mechanismus 41 ist die Blickrichtung 40 einerseits in bezug auf die Längsrichtung des Schaftes gemäß einem Doppelpfeil 42 hin- und herschwenkbar. Die Blickrichtung 40 ist außerdem in Umfangsrichtung gemäß einem Doppelpfeil 34 hin- und herverschwenkbar.

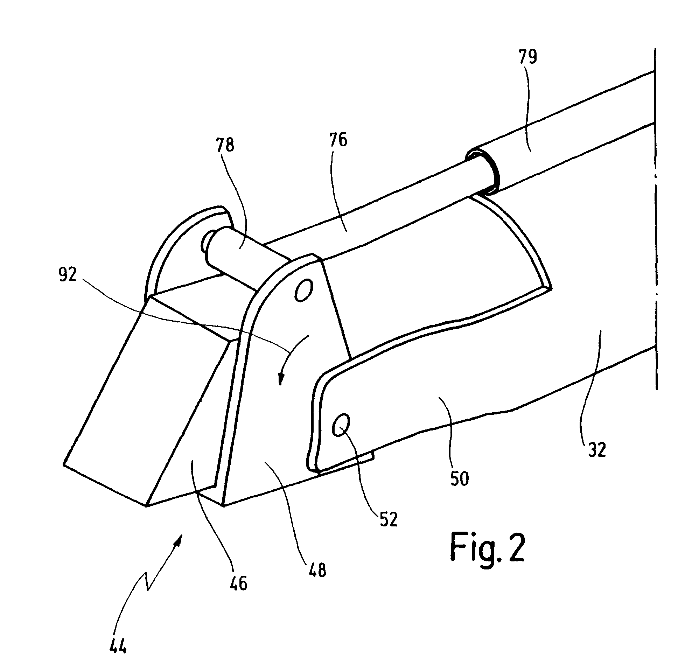

Zur Verschwenkung der Blickrichtung 40 gemäß dem Doppelpfeil 42 weist der Mechanismus 41 ein in dem Außenschaft 12 am distalen Ende 36 angeordnetes lageverstellbares optisches Element 44 auf, das in Fig. 2 in vergrößertem Maßstab dargestellt ist. Das optische Element 44 ist ein Prisma 46, das in einer Fassung 48 befestigt ist, die wiederum zwischen zwei Schenkeln 50 des Innenschaftes 32 um eine Schwenkachse 52, die quer zur Längsrichtung des Schafts 12 verläuft, verschwenkbar gelagert ist. Die Lageverstellung des optischen Elements 44 besteht bei diesem Ausführungsbeispiel demnach in einer Verschwenkung des Prismas 46 um die Schwenkachse 52, wobei die Verschwenkung des optischen Elements 44 der Verschwenkung der Blickrichtung bezüglich der Längsrichtung, d.h. gemäß dem Doppelpfeil 42, dient. Um das optische Element 44 zur Verschwenkung der Blickrichtung 40 in Richtung des Doppelpfeils 42 zwischen einer Vorwärts-Blickrichtung (Blickwinkel von etwa 5° bezüglich der Längsachse des Schafts 12) und einer Rückwärts-Blickrichtung (Blickwinkel von etwa 120°) zu verschwenken, ist am Handgriff 14 ein erstes Bedienelement 54 positioniert, und zwar derart, daß das Bedienelement 54 mit einem Finger, hier geeigneterweise dem Zeigefinger derselben Hand, die den Handgriff 14 umgreift, betätigt werden kann.

Weiterhin umfaßt der Mechanismus 41 zur Verschwenkung der Blickrichtung in Umfangsrichtung eine Verdrehbarkeit des Schafts 12 gemäß dem Doppelpfeil 34.

Um die Blickrichtung 40 in Umfangsrichtung gemäß dem Doppelpfeil 34 zu verschwenken, wobei die Verschwenkung in beiden Richtungen über einen Vollkreis von 360° möglich ist, ist am Handgriff 14 ein zweites Bedienelement 56 derart positioniert, daß das Bedienelement 56 relativ zu dem ersten Bedienelement 54 mit dem Daumen derselben Hand, die den Handgriff 14 hält, betätigt werden kann.

Das erste Bedienelement 54 ist an einer distalen Seite 58 des Handgriffs 14, und zwar bezüglich der Querabmessung des Handgriffs 14 etwa mittig angeordnet. Das erste Bedienelement 54 ist als pistolenartiger Abzug ausgebildet, der gemäß einem Doppelpfeil 62 am Handgriff 14 verschwenkbar gelagert ist, wie noch näher beschrieben wird.

Das erste Bedienelement 54 weist einen Ring 64 auf, der vollumfänglich geschlossen ist und dessen Öffnung im Durchmesser so bemessen ist, daß der Zeigefinger durch den Ring 64 durchgeführt werden kann.

Das zweite Bedienelement 56 ist als Stellrad 66 ausgebildet, das etwa in Höhe des ersten Bedienelements 54, jedoch proximal von diesem beabstandet, am Handgriff 14 positioniert ist.

Der Handgriff 14 weist an einer Seitenfläche 68 ein Fenster 70 auf, in dem ein mit dem Daumen betätigbarer Teilumfang 72 des Stellrades 66 zu liegen kommt.

Auf der der Seitenfläche 68 gegenüberliegenden, in Fig. 1 nicht sichtbaren Seitenfläche der Halbschale 22 des Handgriffs 14 ist ein entsprechendes Fenster vorgesehen, so daß auch auf dieser Seite ein Teilumfang des Stellrades 66 betätigbar zu liegen kommt. Auf diese Weise läßt sich das Stellrad 66 sowohl mit der linken Hand als auch mit der rechten Hand in Ein-Hand-Bedienung jeweils mit dem Daumen betätigen. Die Betätigungsrichtung des Stellrades 66 erfolgt in Richtungen gemäß einem Doppelpfeil 74, d.h. im wesentlichen parallel zur Längsachse des Schafts 12.

Es ergibt sich aus der Darstellung in Fig. 1, daß das erste Bedienelement 54 und das zweite Bedienelement 56 gleichzeitig betätigbar sind, wobei das erste Bedienelement 54 mit beispielsweise dem Zeigefinger und das zweite Bedienelement 56 mit dem Daumen derselben Hand, die den Handgriff 14 hält, betätigt werden kann, und zwar in einer ermüdungsfreien, ergonomischen Handhaltung. Somit kann die Blickrichtung 40 gleichzeitig in Längsrichtung als auch in Umfangsrichtung verschwenkt werden.

Zur Lageverstellung des optischen Elements 44 steht das erste Bedienelement 54 mit diesem in Wirkverbindung, wie nun an Hand von Fig. 2 und 3 näher erläutert wird. Gemäß Fig. 2 ist am oberen Ende der Fassung 48 ein Zugelement 76 über einen Querzapfen 78 angelenkt. Das Zugelement 76 ist durch ein in dem Außenschaft 30 und an dem Innenschaft 32 entlang geführtes Mantelrohr 79 in das Gehäuse 16 geführt, wo es mit seinem anderen Ende an einer Übersetzungshülse 81 befestigt ist (Fig. 3). Die Übersetzungshülse 81 ist mit dem Innenschaft 32 und damit mit dem Schaft 12 insgesamt drehfest verbunden, so daß das Zugelement 76 und das Mantelrohr 79 ebenfalls mit dem Schaft 12 bei einer Drehung mitgedreht werden.

Weiterhin greift in eine etwa mittige Nut 81a in der Übersetzungshülse 81, die demnach aus zwei einstückig miteinander verbundenen beidseits der Nut 81a angeordneten Hülsenhälften 81b und 81c gebildet wird, eine Schubgabel 80 ein, die abgewinkelt, aber starr ausgebildet ist. Der untere Abschnitt der Schubgabel 80 ist in einer Führung 82 gemäß einem Doppelpfeil 83 verschiebbar geführt und steht über einen Querzapfen 84 mit dem ersten Bedienelement 54 in Verbindung, wobei der Querzapfen 84 in eine als Bahnkurve ausgebildete Nut 86 desselben eingreift. Eine Verschwenkung des ersten Bedienelements 54 in Richtung eines Pfeils 88 bewirkt demnach eine Verschiebung des Zugelements 76 in Richtung eines Pfeils 90. Eine Verschiebung des Zugelements 76 in Richtung des Pfeils 90 bewirkt eine Verschwenkung der Fassung 48 um die Schwenkachse 52 und damit des Prismas 46 in Richtung eines Pfeils 92 in Fig. 2, wodurch die Blickrichtung nach vorn verschwenkt wird. Durch umgekehrte Betätigung des ersten Bedienelements 54 entgegen der Richtung des Pfeils 88 wird das Zugelement 76 entgegen der Richtung des Pfeils 90 zum proximalen Ende hin bewegt, wodurch das Prisma 46 entgegen der Richtung des Pfeils 92 verschwenkt wird, wodurch die Blickrichtung zurück verschwenkt wird.

Das zweite Bedienelement 56 in Form des Stellrads 66 ist in dem Handgriff 14 drehbar gelagert, und zwar über eine fest mit dem Stellrad 66 verbundene Welle 94. Die Drehachse des Stellrads 66 verläuft quer zur Längsrichtung des Schafts 12. Die Übertragung der Drehbewegung des Stellrads 66 um seine Drehachse in eine Drehung des Schafts 12 um dessen Längsachse wird durch ein Getriebe 95 in Form eines Kegelradgetriebes bewerkstelligt.

Die Welle 94 trägt dazu an ihrem oberen Ende ein Kegelrad 96, das mit einem fest mit dem Innenschaft 32 verbundenen weiteren Kegelrad 98 kämmt. Eine Drehung des Stellrades 66 um die Längsachse der Welle 94 bewirkt somit eine Drehung des Innenschafts 32 und des fest mit diesem verbundenen Außenschafts 30 um deren gemeinsame Längsmittelachse. Das aus den Kegelrädern 96 und 98 gebildete Getriebe 95 weist gemäß Fig. 3 ein Übersetzungsverhältnis auf, das kleiner als 1 ist, so daß eine Feineinstellung der Blickrichtung in Umfangsrichtung ermöglicht wird.

Wieder mit Bezug auf Fig. 1 ist am Handgriff 14 eine Markierung 100 angebracht, die einen Hinweis darauf gibt, in welcher Richtung das Bedienelement 54 gemäß dem Doppelpfeil 62 betätigt werden muß, um die Blickrichtung 40 nach vorn (5°) oder nach hinten (120°) zu verschwenken.

Der Handgriff 14 ist insgesamt ergonomisch ausgebildet. Dazu ist an der Rückseite des Handgriffs 14 eine Mulde 102 vorgesehen, in der sich die Mulde zwischen Daumen und Zeigefinger der den Handgriff 14 ergreifenden Hand abstützen kann. Entsprechend sind auf der Vorderseite 58 des Handgriffs 14 eine obere Mulde 104 und eine untere Mulde 106 ausgebildet, wobei sich an der oberen Mulde 104 der Mittelfinger und an der unteren Mulde 106 der kleine Finger abstützen kann.

Ferner ist in dem Handgriff 14 ein Anschluß 108 zum Anschließen eines nicht dargestellten lichtführenden Kabels versenkt angeordnet. Auf Höhe des Anschlusses 108 weist der Handgriff 14 beidseitig eine Öffnung 110 auf, die das Anschließen des Licht zuführenden Kabels an dem Anschluß 108 erleichtert.

Am unteren Ende des Handgriffs 14 ist eine sich nach unten hin erweiternde Durchführung 112 für das anzuschließende Licht zuführende Kabel vorgesehen, wobei die Durchführung 112 eine gewisse Biegebeweglichkeit des Kabels zuläßt.

Die Oberfläche des Handgriffs 14 ist insbesondere in seinem von der Hand umschlossenen Griffbereich 114 rauh, um die Griffigkeit zu erhöhen und ein Rutschen des Handgriffs 14 in der Hand zu vermeiden.

Eine Aufbewahrungsöffnung 116 dient zum unverlierbaren Verstauen einer nicht dargestellten Schutzkappe für das distale Ende 36 des Schafts 12 während der Benutzung des Endoskopes 10.

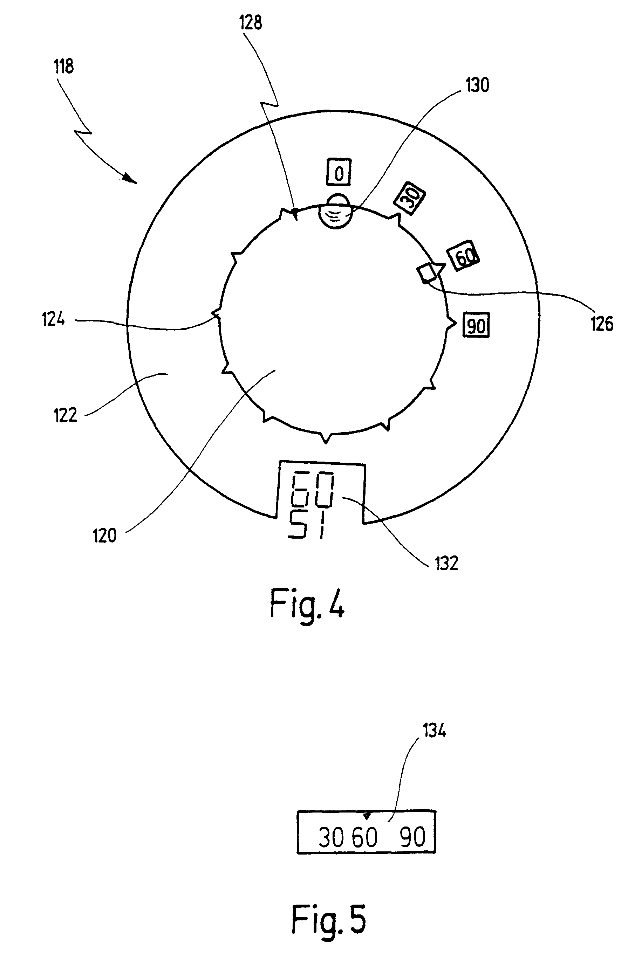

Das Endoskop 10 weist ferner eine Anzeigeeinrichtung 118 für die jeweils eingestellte Blickrichtung auf, die, wie in Fig. 4 dargestellt ist, in einem Sucherbild 120 beim Durchblicken durch die Endoskopoptik sichtbar ist. Die Anzeigeeinrichtung 118 umfaßt ein lochscheibenförmiges Element 122, das umfänglich verteilt Referenzmarkierungen 124 aufweist, die in dem gezeigten Ausführungsbeispiel in Winkelabständen von 30° beabstandet sind. Relativ zu dem lochscheibenförmigen Element 122 drehbar weist die Anzeigeeinrichtung 118 einen Zeiger 126 auf, dessen Stellung die jeweilig eingestellte Blickrichtung 40 in Umfangsrichtung anzeigt. An den Referenzmarkierungen 124 können, wie in Fig. 4 dargestellt ist, Beschriftungen angebracht sein, die eine grobe Abschätzung der eingestellten Blickrichtung 40 in Umfangsrichtung im Zusammenwirken mit dem Zeiger 126 ermöglichen. Die 0°-Richtung entspricht dabei der Gegenrichtung des Handgriffs 14. Bei vertikal gehaltenem Handgriff 14 ermöglicht es die Anzeigeeinrichtung 118 somit, an Hand des Zeigers 126 den Winkel der Blickrichtung 40 in Umfangsrichtung in bezug auf die Vertikale bzw. Lotrichtung abzulesen und dadurch bspw. die Lage eines unter dieser Blickrichtung "gepeilten" Beobachtungsobjekts im Beobachtungsraum zu ermitteln und zu dokumentieren.

Die Anzeigeeinrichtung 118 kann so aufgebaut sein, daß das lochscheibenförmige Element 122 eine Blende ist, die relativ zum Gehäuse 16 des Endoskops 10 fest ist, während der Zeiger 126 beim Drehen des Schafts 12 von diesem mitgedreht wird, oder umgekehrt.

Ferner weist die Anzeigeeinrichtung 118 zusätzlich eine Flüssigkeitslibelle 128 auf, indem in einer Flüssigkeit eine Gasblase 130 wie bei einer Wasserwaage vorhanden ist. Dies dient als weitere Orientierung des Benutzers, da die Glasblase 130 stets die Lotrichtung anzeigt, d.h. auch dann, wenn der Handgriff 114 nicht streng vertikal, sondern schräg gehalten wird.

Ferner weist die Anzeigeeinrichtung 118 noch eine Digitalanzeige auf, die ebenfalls im Sucherbild 120 sichtbar ist. Die Digitalanzeige umfaßt eine Anzeige der Blickrichtung 40 in Umfangsrichtung (obere Zahl) und eine Anzeige der Blickrichtung 40 in Längsrichtung (untere Zahl), die jeweils den Winkel der eingestellten Blickrichtung bezogen auf die Vertikale bzw. bezogen auf die Längsrichtung des Schafts 12 in Grad anzeigen.

Alternativ zu der Digitalanzeige 132 in Fig. 4 ist in Fig. 5 eine Analoganzeige 134 dargestellt, die im Sucherbild 120 sichtbar ist.

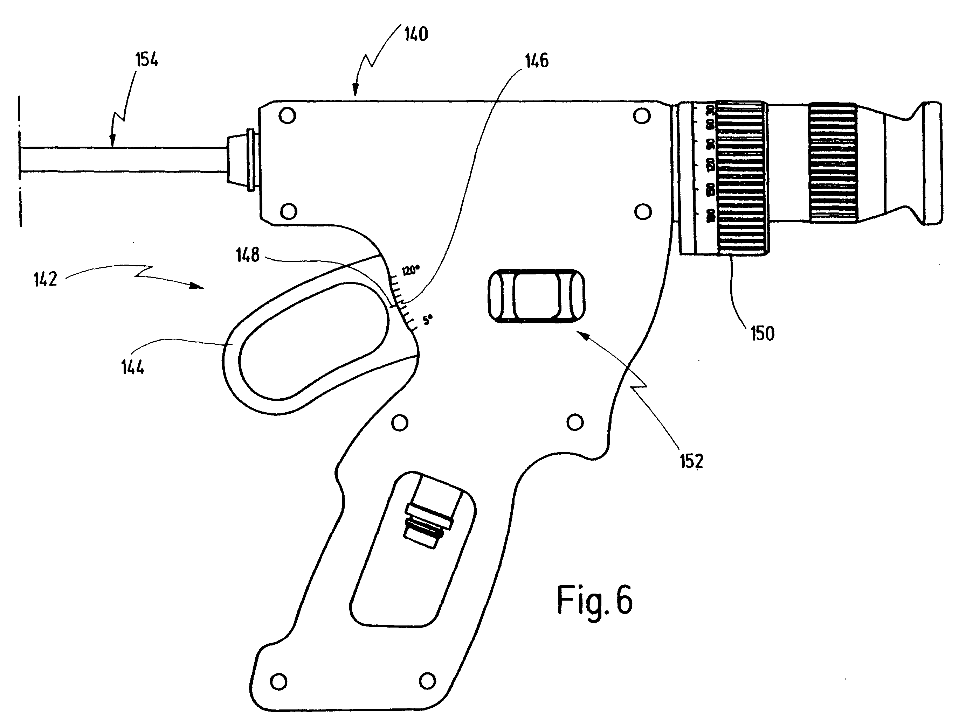

In Fig. 6 ist gemäß einem weiteren Ausführungsbeispiel ein gegenüber dem Endoskop 10 in Figuren 1 bis 3 abgewandeltes Endoskop 140 dargestellt. Ein Bedienelement 142 zur Verschwenkung der Blickrichtung in Längsrichtung weist einen Ring 144 auf, der ebenfalls umfänglich vollständig geschlossen ist, der jedoch größer dimensioniert ist als der Ring 64 in Fig. 1, so daß der Ring 144 zur Bedienung mit zwei Fingern, Zeige- und Mittelfinger, geeignet ist.

Ferner ist eine Markierung 146 vorgesehen, die Richtungsmarkierungen für die Verstellung der Blickrichtung in Längsrichtung aufweist, die mit einer Markierung 148 am Ring 144 zusammenwirken.

Ferner weist das Endoskop 140 einen Drehring 150 auf, der wie ein Bedienelement 152 zum Verdrehen eines Schafts 154 des Endoskops 140 dient, wobei der Drehring 150 eine Schnellverdrehung über einen größeren Drehbereich ermöglicht, während mit dem Bedienelement 152 in Form eines Stellrades eine Feinverdrehung des Schafts 154 und damit eine Feinverschwenkung der Blickrichtung in Umfangsrichtung ermöglicht wird.

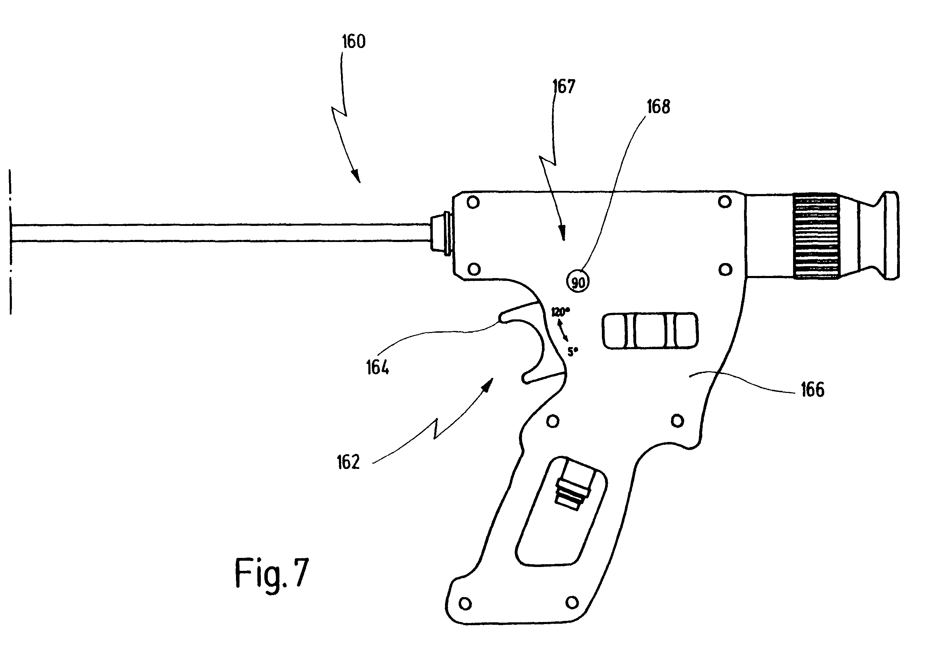

In Fig. 7 ist schließlich noch ein weiteres Ausführungsbeispiel eines Endoskops 160 dargestellt. Das Endoskop 160 unterscheidet sich von dem Endoskop 140 und dem Endoskop 10 durch ein Bedienelement 162 zur Verschwenkung der Blickrichtung bzgl. der Längsrichtung, das einen teilweise offenen Ring 164 in Form einer Wippe aufweist.

Ferner ist an einem Handgriff 166 des Endoskops 160 eine Anzeigeeinrichtung 167 vorgesehen, die ein Anzeigefenster 168 aufweist, in dem eine Digital- oder Analoganzeige für die eingestellte Blickrichtung sichtbar ist. Es können auch zwei derartige Anzeigefenster 168 vorgesehen sein, und zwar eines für die Anzeige der Blickrichtung in Umfangsrichtung, und eines für die Anzeige der Blickrichtung in Längsrichtung.

## Patentansprüche

1. Endoskop, mit einem Schaft (12), mit einem proximal angeordneten seitlich von dem Schaft (12) abstehenden pistolenartigen Handgriff (14), mit einem Mechanismus (41) zur Verschwenkung einer Blickrichtung (40) in einem Beobachtungsraum, und mit zumindest zwei mit dem Mechanismus (41) in Wirkverbindung stehenden Bedienelementen (54, 56; 142; 152; 162) zur Betätigung des Mechanismus (41), wobei das zumindest eine Bedienelement (54; 142; 162) zur Verschwenkung der Blickrichtung (40) bezüglich der Längsrichtung des Schafts (12) und das zumindest eine weitere Bedienelement (56; 152) zur Verschwenkung der Blickrichtung (40) in Umfangsrichtung des Schafts (12) dient, wobei das eine Bedienelement (52; 142; 162) an einer Vorderseite des Handgriffs (14) so angeordnet ist, daß es mit zumindest einem Finger der Hand, die den Handgriff (14) hält, betätigbar ist, **dadurch gekennzeichnet, daß** das zumindest eine andere Bedienelement (56; 152) mit dem Daumen derselben Hand betätigbar an einer Seitenfläche (68) des Handgriffs (14) angeordnet ist.

2. Endoskop nach Anspruch 1, **dadurch gekennzeichnet, daß** das zumindest eine Bedienelement (54; 142; 162) als pistolenartiger Abzug ausgebildet.

3. Endoskop nach Anspruch 1, **dadurch gekennzeichnet, daß** das zumindest eine Bedienelement als Taste, is Tastenpaar oder als Wipptaste ausgebildet ist.

4. Endoskop nach Anspruch 1 oder 2, **dadurch gekennzeichnet, daß** das zumindest eine Bedienelement (54; 142; 162) einen umfänglich geschlossenen oder umfänglich teilweise offenen Ring (64; 144; 164) zum Durchführen oder Anlegen eines oder mehrerer Finger aufweist.

5. Endoskop nach Anspruch 1, **dadurch gekennzeichnet, daß** das zumindest eine andere Bedienelement als Schieber ausgebildet ist, der an der zumindest einen Seitenfläche des Handgriffs angeordnet ist.

6. Endoskop nach einem der Ansprüche 1 bis 5, **dadurch gekennzeichnet, daß** das zumindest eine andere Bedienelement (56; 152) als Stellrad (66) ausgebildet ist, das in dem Handgriff (14) drehbar gelagert ist, wobei der Handgriff (14) auf zumindest einer seiner Seitenflächen (68) ein Fenster (70) aufweist, in dem ein Teilumfang des Stellrads (66) zu liegen kommt.

7. Endoskop nach Anspruch 6, **dadurch gekennzeichnet, daß** das Stellrad (66) mit nicht parallel zur Längsachse des Schafts (12) verlaufender Drehachse in dem Handgriff (14) angeordnet ist.

8. Endoskop nach einem der Ansprüche 1 bis 7, **dadurch gekennzeichnet, daß** der Mechanismus (41) zur Verschwenkung der Blickrichtung zumindest ein am distalen Ende (36) des Schafts (12) lageverstellbar angeordnetes optisches Element (44) aufweist, wobei die Lageverstellung des optischen Elements (44) der Verschwenkung der Blickrichtung (40) im wesentlichen bezüglich der Längsrichtung des Schafts (12) dient, und wobei das zumindest eine Bedienelement (54; 142; 162) mit dem optischen Element (44) in Wirkverbindung steht.

9. Endoskop nach einem der Ansprüche 1 bis 8, **dadurch gekennzeichnet, daß** der Mechanismus (41) eine Verdrehbarkeit des Schafts (12) umfaßt, wobei der Schaft (12) zur Verschwenkung der Blickrichtung (40) in Umfangsrichtung des Schafts (12) um seine Längsachse drehbar ist, wobei das zumindest eine andere Bedienelement (56; 152) mit dem Schaft (12) zum Drehen des Schafts (12) in Wirkverbindung steht.

10. Endoskop nach einem der Ansprüche 1 bis 9, **dadurch gekennzeichnet, daß** das zumindest eine und/oder das zumindest eine weitere Bedienelement (54, 56; 142; 152; 162) selbsthemmend ausgebildet ist.

11. Endoskop nach einem der Ansprüche 1 bis 10, **dadurch gekennzeichnet, daß** das zumindest eine und/oder das zumindest eine weitere Bedienelement (54, 56; 142; 152; 162) in zumindest einer Betätigungsstellung arretierbar ist.

12. Endoskop nach einem der Ansprüche 1 bis 11, **dadurch gekennzeichnet, daß** zumindest eine Anzeigeeinrichtung (118; 167) zur Anzeige der jeweils eingestellten Blickrichtung (40) vorgesehen ist.

13. Endoskop nach Anspruch 12, **dadurch gekennzeichnet, daß** die Anzeigeeinrichtung (118) im Bildübertragungsweg des Endoskops (10; 140; 160) angeordnet ist, so daß die eingestellte Blickrichtung (40) in einem Sucherbild (120) angezeigt wird.

14. Endoskop nach Anspruch 13, **dadurch gekennzeichnet, daß** die Anzeigeeinrichtung (118) ein lochscheibenförmiges Element (122), das umfänglich verteilt Referenzmarkierungen (124) aufweist, und einen relativ zu diesen um die Längsachse des Schafts umlaufenden Zeiger (126) umfaßt.

15. Endoskop nach Anspruch 13 oder 14, **dadurch gekennzeichnet, daß** die Anzeigeeinrichtung (118) eine Flüssigkeitslibelle (128) zur Anzeige der Lotrichtung umfaßt.

16. Endoskop nach einem der Ansprüche 12 bis 15, **dadurch gekennzeichnet, daß** die Anzeigeeinrichtung (118) eine Digital- oder Analoganzeige (132; 134) für die eingestellte Blickrichtung (40) umfaßt.

17. Endoskop nach einem der Ansprüche 12 bis 16, **dadurch gekennzeichnet, daß** die Anzeigeeinrichtung (167) ein oder mehrere am Handgriff von außen sichtbar angeordnete Anzeigefenster (168) mit einer Digital- oder Analoganzeige für die eingestellte Blickrichtung (40) umfaßt.

18. Endoskop nach einem der Ansprüche 1 bis 17, **dadurch gekennzeichnet, daß** der Handgriff (14) vom Schaft (12) zum distalen Ende (36) hin geneigt absteht.

19. Endoskop nach einem der Ansprüche 1 bis 18, **dadurch gekennzeichnet, daß** im Handgriff (14) versenkt ein Anschluß (108) zum Anschließen eines lichtzuführenden Kabels angeordnet ist, wobei der Handgriff (14) in Höhe des Anschlusses (108) auf zumindest einer Seite eine Öffnung (110) aufweist.

20. Endoskop nach einem der Ansprüche 1 bis 19, **dadurch gekennzeichnet, daß** am proximalen Ende des Endoskops (10) eine TV-Kamera integriert ist.

21. Verwendung eines Endoskops nach einem der Ansprüche 1 bis 20 in technischen Anwendungen, insbesondere in der Untersuchung von Brennräumen.

## Claims

1. An endoscope, comprising a shaft (12), a proximally arranged pistol-like handle (14) which laterally protrudes from the shaft (12), a mechanism (41) for pivoting a viewing direction (40) in an observation area, and at least two operating elements (54, 56; 142; 152; 162) for operating the mechanism (41) which are operatively connected with the mechanism (41), wherein the at least one operating elment (54; 142; 162) serves the pivoting of the viewing direction (40) with respect to the longitudinal direction of the shaft (12) and the at least one further operating element (56; 152) serves the pivoting of the viewing direction (40) in circumferential direction of the shaft (12), wherein the one operating element (52; 142; 162) is arranged on a front side of the handle (14) such that it is operable by at least one finger of that hand which holds the handle (14), **characterized in that** the at least one other operating element (56; 152) is arranged on a side face (68) of the handle (14) and is operable by the thumb of the same hand.

2. The endoscope of claim 1, **characterized in that** the at least one operating element (54; 142; 162) is configured as a pistol-like trigger.

3. The endoscope of claim 1, **characterized in that** the at least one operating element is configured as a button, a pair of buttons or as a toggle switch.

4. The endoscope of claim 1 or 2, **characterized in that** the at least one operating element (54; 142; 162) comprises a ring (64; 144; 164) closed or partially open at its circumference for passing or laying on one or more fingers.

5. The endoscope of claim 1, **characterized in that** the at least one other operating element is configured as a slider which is arranged on the at least one side face of the handle.

6. The endoscope of anyone of claims 1 through 5, **characterized in that** the at least one other operating element (56; 152) is configured as an adjustment wheel (66) rotatably mounted in the handle (14), wherein the handle (14) comprises a window (70) on at least one of its side faces (68), in which a partial circumference of the adjustment wheel (66) is exposed.

7. The endoscope of claim 6, **characterized in that** the adjustment wheel (66) is arranged in the handle (14) with its pivot axis not being parallel to the longitudinal axis of the shaft (12).

8. The endoscope of anyone of claims 1 through 7, **characterized in that** the mechanism (41) for pivoting the view direction comprises at least one optical element (44) arranged to be adjustable in position at the distal end (36) of the shaft (12), wherein the position adjustment of the optical element (44) serves the pivoting of the viewing direction (40) substantially with respect to the longitudinal direction of the shaft (12), and wherein the at least one operating element (54; 142; 162) is operatively connected with the optical element (44).

9. The endoscope of anyone of claims 1 through 8, **characterized in that** the mechanism (41) includes a rotatability of the shaft (12), wherein the shaft (12) is rotatable about its longitudinal axis to pivot the viewing direction (40) in circumferential direction of the shaft (12), and wherein the at least one other operating element (56; 112) is operatively connected with the shaft (12) for rotating the shaft (12).

10. The endoscope of anyone of claims 1 through 9, **characterized in that** the at least one and/or the at least one further operating element (54, 56; 142; 152; 162) are configured to be self-locking.

11. The endoscope of anyone of claims 1 through 10, **characterized in that** the at least one and/or the at least one further operating element (54, 56; 142; 152; 162) are lockable in at least one operating position.

12. The endoscope of anyone of claims 1 through 11, **characterized in that** at least one display device (118; 167) is provided for displaying the respectively adjusted viewing direction (40).

13. The endoscope of claim 12, **characterized in that** the display device (118) is arranged in the image transmission path of the endoscope (10; 140; 160), so that the adjusted viewing direction (40) is displayed in a view finder field (120).

14. The endoscope of claim 13, **characterized in that** the display device (118) comprises an apertured-disc-like element (122) having reference markings (124) distributed about its circumference and a pointer (126) running about the longitudinal axis of the shaft relative to the markings.

15. The endoscope of claim 13 or 14, **characterized in that** the display device (118) comprises a bubble in a fluid (128) for indicating the vertical direction.

16. The endoscope of anyone of claims 12 through 15, **characterized in that** the display device (118) comprises a digital or analog display (132; 134) for the adjusted viewing direction (40).

17. The endoscope of anyone of claims 12 through 16, **characterized in that** the display device (167) comprises one or more display windows (168) arranged on the handle to be visible from the outside with a digital or analog display for the adjusted viewing direction (40).

18. The endoscope of anyone of the claims 1 through 17, **characterized in that** the handle (14) protrudes from the shaft (12) to be inclined toward the distal end (36).

19. The endoscope of anyone of claims 1 through 18, **characterized in that** a connector (108) is arranged to be recessed in the handle (14) for connecting a light supplying cable, wherein the handle (14) comprises an opening (110) on at least one side on a level with the connector (108).

20. The endoscope of anyone of claims 1 through 19, **characterized in that** a TV camera is integrated at the proximal end of the endoscope (10).

21. Use of an endoscope according to anyone of claims 1 through 20 in technical applications, in particular in the investigation of combustion chambers.

## Revendications

1. Endoscope avec une tige (12), avec une poignée de type pistolet (14) placée écartée de la tige (12) de manière proximale sur le côté, avec un mécanisme (41) pour le pivotement d'une direction de prise de vue (40) dans une chambre d'observation, et avec au moins deux éléments de service (54, 56 ; 142 ; 152 ; 162) en relation d'action avec le mécanisme (41) pour l'actionnement du mécanisme (41), le au moins un élément de service (52 ; 142 ; 162) pour le pivotement de la direction de prise de vue (40) fonctionnant selon la direction longitudinale de la tige (12) et le au moins un autre élément de service (56 ; 152) pour le pivotement de la prise de vue (40) fonctionnant en direction de la circonférence de la tige (12), l'élément de service (52 ; 142 ; 152) étant placé sur un côté avant de la poignée (14), de telle sorte qu'il puisse être actionné avec au moins un doigt de la main qui tient la poignée (14), **caractérisé en ce qu'**au moins un autre élément de service (56 ; 152) pouvant être actionné avec le pouce de la même main est placé sur une surface latérale (68) de la poignée (14).

2. Endoscope selon la revendication 1, **caractérisé en ce qu'**au moins un élément de service (54 ; 142 ; 162) est formé comme une détente de pistolet.

3. Endoscope selon la revendication 1, **caractérisé en ce que** le au moins un élément de service est formé comme une touche, comme une paire de touches ou comme une touche à bascule.

4. Endoscope selon la revendication 1 ou 2, **caractérisé en ce que** le au moins un élément de service (54 ; 142 ; 162) présente un anneau fermé en circonférence ou partiellement ouvert en circonférence (64 ; 144 ; 164) pour l'introduction ou le placement d'un ou plusieurs doigts.

5. Endoscope selon la revendication 1, **caractérisé en ce qu'**au moins un autre élément de service est formé comme un curseur qui est placé sur le au moins un côté latéral de la poignée.

6. Endoscope selon l'une des revendications 1 à 5, **caractérisé en ce que** le au moins un autre élément de service (56 ; 152) est formé comme une roue de commande (66), placée de manière à pouvoir être tirée dans la poignée (14), la poignée (14) présentant sur au moins l'une de ses surfaces latérales (68) une fenêtre (70) dans laquelle s'appuie une partie de la circonférence de la roue de commande (66).

7. Endoscope selon la revendication 6, **caractérisé en ce que** la roue de commande (66) est dotée dans la poignée (14) d'un axe de rotation non parallèle à l'axe longitudinal de la tige (12).

8. Endoscope selon l'une des revendications 1 à 7, **caractérisé en ce que** le mécanisme (41) de pivotement de la direction de prise de vue présente au moins un élément optique (44) placé de manière positionnable à l'extrémité distale (36) de la tige (12), le positionnement de l'élément optique (44) servant au pivotement de la direction de prise de vue (40) dans l'ensemble selon la direction longitudinale de la tige (12) et le au moins un élément de service (54 ; 142 ; 162) étant en relation d'action avec l'élément optique (44).

9. Endoscope selon l'une des revendications 1 à 8, **caractérisé en ce que** le mécanisme (41) comprend une possibilité de rotation de la tige (12), la tige (12) pouvant tourner pour le pivotement de la direction de prise de vue (40) en direction de circonférence de la tige (12) autour de son axe longitudinal, le au moins un autre élément de service (56 ; 152) étant en relation d'action avec la tige (12) pour faire tourner la tige (12).

10. Endoscope selon l'une des revendications 1 à 9, **caractérisé en ce que** le au moins un et/ou le au moins un autre élément de service (54, 56 ; 142 ; 152 ; 162) sont conçus comme pouvant se bloquer automatiquement.

11. Endoscope selon l'une des revendications 1 à 10, **caractérisé en ce que** le au moins un et/ou le au moins un autre élément de service (54, 56 ; 142 ; 152 ; 162) peuvent être bloqués en au moins une position d'actionnement.

12. Endoscope selon l'une des revendications 1 à 11, **caractérisé en ce qu'**au moins un dispositif d'affichage (118; 167) est prévu pour l'affichage de la direction de prise de vue (40) établie.

13. Endoscope selon la revendication 12, **caractérisé en ce que** le dispositif d'affichage (118) est placé sur le chemin de transmission d'image de l'endoscope (10 ; 140 ; 160), de telle sorte que la direction de point de vue (40) établie soit affichée dans une image de viseur (120).

14. Endoscope selon la revendication 13, **caractérisé en ce que** le dispositif d'affichage (118) comprend un élément en forme de disque perforé (122) qui présente des marques de référence (124) réparties sur sa circonférence, et un pointeur (126) s'étendant par rapport à celui-ci sur l'axe longitudinal de la tige.

15. Endoscope selon la revendication 13 ou 14, **caractérisé en ce que** le dispositif d'affichage (118) comprend un niveau de liquide à bulle (128) pour afficher la direction perpendiculaire.

16. Endoscope selon l'une des revendications 12 à 15, **caractérisé en ce que** le dispositif d'affichage (118) comprend un écran numérique ou analogique (132 ; 134) pour la direction de point de vue (40) établie.

17. Endoscope selon l'une des revendications 12 à 16, **caractérisé en ce que** le dispositif d'affichage (167) comprend une ou plusieurs fenêtres d'affichage (168) placées sur la poignée et visibles de l'extérieur avec un écran numérique ou analogique pour la direction de point de vue (40) établie.

18. Endoscope selon l'une des revendications 1 à 17, **caractérisé en ce que** la poignée (14) s'écarte de la tige (12) en étant inclinée vers l'extrémité distale (36).

19. Endoscope selon l'une des revendications 1 à 18, **caractérisé en ce que**, enfoncé dans la poignée (14), se trouve un raccordement (108) pour raccorder un câble transmettant la lumière, la poignée (14) présentant une ouverture (110) à hauteur du raccordement (108) au moins sur un côté.

20. Endoscope selon l'une des revendications 1 à 19, **caractérisé en ce que**, à l'extrémité proximale de l'endoscope, est intégrée une caméra TV.

21. Utilisation d'un endoscope selon l'une des revendications 1 à 20 dans des utilisations techniques, en particulier pour l'étude de chambres de combustion.
